# EUROPEAN PATENT APPLICATION

(11) **EP 4 600 268 A1**
(43) Date of publication of application: **13.08.2025**
(21) Application number: 23874311.6
(22) Date of filing: 28.09.2023
(51) Int. Cl.: C07K 14/78, A61L 27/24, A61P 19/00

(54) **RECOMBINANT COLLAGEN AND USE THEREOF IN CARTILAGE REPAIR MATRIX**

(30) Priority: 08.10.2022 CN 202211224593; 04.11.2022 CN 202211378516
(71) Applicant: Shanxi Jinbo Biopharmaceutical Co., Ltd., Taiyuan, Shanxi 030032 (CN); Sichuan University, Chengdu, Sichuan 610065 (CN)
(72) Inventor: LIN, Hai, Chengdu, Sichuan 610065 (CN); XU, Yang, Chengdu, Sichuan 610065 (CN); WANG, Jing, Chengdu, Sichuan 610065 (CN); LIU, Zhanhong, Chengdu, Sichuan 610065 (CN); YANG, Xia, Taiyuan, Shanxi 030032 (CN); ZHANG, Xingdong, Chengdu, Sichuan 610065 (CN)
(74) Representative: Ipey
(86) International application number: PCT/CN2023/122682
(87) International publication number: WO 2024/074119

(57) **Abstract**

The present invention discloses a recombinant collagen protein and its use in the cartilage repair matrix. The present invention discloses a recombinant collagen protein which contains the sequence shown in SEQ ID No. 1; the amino acid sequence of the stated recombinant collagen protein comprises N basic repetitive units; the basic repetitive unit contains n1 amino acid sequences with the following characteristic: G-Xaa₁-Xaa₂-G-E-Xaa₃; the 3' end and the 5' end of the basic repetitive unit are connected to form the amino acid sequence with the above characteristic. The recombinant collagen protein claimed by the present invention has significant integrin binding activity, has the effect of promoting cell adhesion, proliferation, differentiation, and repairing cartilage tissue defects, etc, and it has promising prospects for application.

## Description

This application claims priority to (1) an application filed with the China National Intellectual Property Administration (CNIPA) on October 8, 2022, with application number 2022112245930, and (2) an application filed with the China National Intellectual Property Administration (CNIPA) on November 4, 2022, with application number 2022113785160. The entire contents of the prior applications are incorporated herein by reference.

### Technical Field

The present invention belongs to the field of biomaterials, in particular to a recombinant collagen protein and its use in cartilage repair matrix.

### Background Technology

Articular cartilage injury has become a common disease in orthopedics, which may be induced by trauma, arthritis, and sports-related injuries. The repair of articular cartilage mainly depends on the proliferation and differentiation of chondrocytes which produce sufficient extracellular matrix to repair cartilage defects. However, chondrocytes in healthy adults are usually resting and highly-differentiated cells. Due to their low vascularization, they are mainly nourished by joint fluid and subchondral bone. The regeneration of articular cartilage is very limited in the case of traumatic or pathological injury. Currently, some of the treatment methods used in the clinic are microfracture, osteochondral transplantation, chondrocyte transplantation, etc. These methods can repair cartilage defects to a certain extent and alleviate the pain of the patients, but the long-term results are not satisfactory. The main reason is that the cartilage reconstructed by these methods is organizationally and structurally different from the normal hyaline cartilage, which leads to the disparity in terms of histological and biomechanical properties.

In recent years, tissue engineering has made considerable progress in functional articular cartilage, and a number of bioengineered therapeutic strategies including stem cell approaches and scaffolding technologies have been proposed to provide minimally invasive and more effective ways to repair articular cartilage. Scaffolding materials, as one of the three major elements of tissue engineering, play an important role in the construction of tissue-engineered cartilage, and their mechanical properties and chemical composition will influence cell adhesion and proliferation, and cellular phenotype, and among others. Collagen, with its excellent biocompatibility, bioactivity, and biodegradability, has become a more studied biomaterial for tissue engineering. However, most of the traditional collagen is derived from animals, and the amino acid sequence of animal-derived collagen has a high degree of similarity to that of human collagen, such as 95% similarity between mammalian pigs and cows and 65% similarity between fish and humans. However, when animal-derived collagen is used in biomaterials or medical devices, these remained differences may pose obvious risks of immunological rejection, sensitization response, and viral infection and transmission. At the same time, since different subtypes of natural collagen are unevenly distributed and coexist in composite form in tissues and organs, the efficiency of collagen extraction from biological tissues depends largely on its natural abundance thereof, and it is also difficult to avoid the mixing of different subtypes. Therefore, the deficiencies in batch stability, purity, molecular weight and distribution of animal-derived collagen further limit its many applications in biomedical fields.

In order to solve the above issues, researchers have focused their attention on the preparation of recombinant collagen protein using genetic engineering techniques. Recombinant collagen protein is a recombinant protein material based on the natural collagen gene sequence of human beings, specifically, the codons with specific functional expression are selected and transcribed into host cells after appropriate modification and editing, then biotechnological fermentation techniques are employed to achieve large-scale production of the recombinant protein material.

### Content of the Invention

The technical issue to be solved by the present invention is to provide a recombinant collagen protein and its use in cartilage repair matrix for the insufficiency of animal-derived collagen, and the provided recombinant collagen protein has more obvious integrin binding activity, which can promote cell adhesion, proliferation, differentiation, and has the effect of repairing cartilage tissue defects.

Integrin binding activity is related to the integrin family, a group of widely distributed transmembrane glycoproteins that are the link between the extracellular matrix and intracellular signaling. Integrins is a heterodimer formed by the connection of α- and β-subunits through non-covalent bonds. A total of 18 α-subunits and 8 β-subunits have been identified, which can be combined to form at least 24 integrin heterodimers. The integrin family of cell adhesion molecules is the main connecting substance between the extracellular matrix and parenchymal cells, such as inflammatory cell and fibroblasts, and is closely related to cell adhesion, spreading, migration, functional expression, and the onset, maintenance, and development of tissue fibrosis. It can regulate interactions such as recognition and adhesion between cells and between cells and the extracellular matrix.

The first purpose of the present invention is to provide a recombinant collagen protein, characterized in that the amino acid sequence of recombinant collagen protein comprises N basic repetitive units and, the stated basic repetitive unit comprises n1 amino acid sequences with the following characteristic: "G-Xaa₁-Xaa₂-G-E-Xaa₃"; the 3' end and the 5' end of the basic repetitive unit are connected to form the amino acid sequence with the above characteristic;

Wherein, N is an integer greater than 4; n1 is an integer greater than 3.

Further, the N value is an integer within 4 to 300, and further an integer within 4 to 200.

The stated N value can make the molecular weight of the recombinant collagen protein reach 1 kDa to 250 kDa, and further reach 3 kDa to 150 kDa.

Further, the characteristic amino acid sequences are arranged consecutively or at intervals in basic repetitive units.

Further, the stated amino acid sequences of the stated recombinant collagen protein exhibit the following characteristics:

[-G-E- Xaa3-Yaa1-(G-Xaa1-Xaa2-G-E-Xaa3)n2-Yaa2-(G-Xaa1-Xaa2 -G-E-Xaa3)n3-Yaa3-
(G-Xaa1-Xaa2-G-E-Xaa3)n3-Yaa4-(G-Xaa1-Xaa2-G-E-Xaa3)n4-…-Yaan-(G-Xaa1-Xaa2-
G-E-Xaa3)n-Yaan+1-G-Xaa1-Xaa2-]N

Wherein, the stated Xaa₁ is a non-polar hydrophobic amino acid; the stated Xaa₂ is one of serine (S), alanine (A), proline (P), and oxyproline (O); and the stated Xaa₃ is a basic amino acid;
Each occurrence of Yaa₁, Yaa₂, Yaa₃, Yaa₄, ..., Yaaₙ, Yaaₙ₊₁ is independently selected from none, one or more different or identical amino acids;
n2, n3, n4, ..., n all are an integer above 0, but they are not 0 at the same time.

The non-polar hydrophobic amino acids described in the present invention include one of glycine (G), valine (V), phenylalanine (F), alanine (A), leucine (L), isoleucine (I), methionine (M), proline (P), and oxyproline (O); and further include phenylalanine (F), alanine (A), leucine (L), isoleucine (I), methionine (M), proline (P), and oxyproline (O).

The stated basic amino acid comprises one of lysine, arginine, and histidine, and further is lysine or arginine.

Further, the stated recombinant collagen protein sequence does not contain protein tags.

In an example of the present invention, the amino acid sequence of the recombinant collagen protein as described in the present invention is shown in SEQID NO. 1.

The second purpose of the present invention is to provide a preparation method of recombinant collagen protein as described in the present invention, characterized in that the recombinant collagen protein is obtained through host cell expression, extraction and purification using gene recombination technology; the amino acid sequence of the stated recombinant collagen protein is obtained by repeated connection of basic repetitive units with multiple repetitions;
Further, the amino acid sequence of the stated recombinant collagen protein comprises N basic repetitive units and, the stated basic repetitive unit comprises n1 amino acid sequences with the following characteristic: "G-Xaa₁-Xaa₂-G-E-Xaa₃"; the 3' end and the 5' end of the basic repetitive unit are connected to form the amino acid sequence with the above characteristic;
Wherein, N is an integer greater than 4; n1 is an integer greater than 3;
Further, the characteristic amino acid sequences are arranged consecutively or at intervals in the basic repetitive units.

Therefore, the N value is an integer from 4 to 300, and further an integer from 4 to 200; including, but not limited to, 4, 5, 6, 7, 8, 9, 10, 11, 12, 13, 14, 15, 16, 17, 18, 19, 20, 21, 22, 23, 24, 25, 26, 27, 28, 29, 30, ..., 200.

Further, the stated N value can make the molecular weight of the recombinant collagen protein reach 1 kDa to 200 kDa, and further reach 3 kDa to 150 kDa.

The N value may be taken as the two endpoint values defined by the present invention, or as any integer in the range value, e.g., the N value may be 1, 3, 10, 50, 70, 90, 95, 100, 125, 150, etc.

Further, the stated recombinant collagen protein sequence does not contain protein tags;
The amino acid sequence of the stated recombinant collagen exhibits the following characteristics:

[-G-E-Xaa3-Yaa1-(G-Xaa1-Xaa2-G-E-Xaa3)n2-Yaa2-(G-Xaa1-Xaa2-G-E-Xaa3)n3-Yaa3-
(G-Xaa1-Xaa2-G-E-Xaa3)n3-Yaa4-(G-Xaa1-Xaa2-G-E-Xaa3)n4-…-Yaan-(G-Xaa1-Xaa2-
G-E-Xaa3)n-Yaan+1-G-Xaa1-Xaa2-]N

Wherein, the stated Xaa₁ is a non-polar hydrophobic amino acid; the stated Xaa₂ is one of serine (S), alanine (A), proline (P), and oxyproline (O); the stated Xaa₃ is a basic amino acid;
Each occurrence of Yaa₁, Yaa₂, Yaa₃, Yaa₄, ..., Yaaₙ, Yaaₙ₊₁ is independently selected from none, one or more different or identical amino acids;
n2, n3, n4, ..., n all are an integer above 0, but they are not 0 at the same time.

The third purpose of the present invention is to provide the use of the above-mentioned recombinant collagen protein in the preparation of products for regulating cell adhesion, proliferation, and differentiation.

The fourth purpose of the present invention is to provide the use of the above-mentioned recombinant collagen protein in the preparation of products for cartilage repair matrix.

In the application of the recombinant collagen protein stated in the present invention, it can be used in combination with polymers and/or their derivatives. The polymers include natural polymers and synthetic polymers; further, the stated natural polymers include, but are not limited to, hyaluronic acid, chitosan, alginic acid, cellulose, etc; further, the stated synthetic polymers include, but are not limited to, PLA, PGA, PLCL, and PVA.

In the application of the recombinant collagen protein stated in the present invention, it is used at a concentration ranging from 0.01 to 100 mg/mL, for example, it can be 0.01 mg/mL, 0.05 mg/mL, 2 mg/mL, 4.5 mg/mL, 7 mg/mL, 10 mg/mL, 15 mg/mL, 20 mg/mL, 50 mg/mL, 80 mg/mL, 100mg/mL, etc. The specific concentration used depends on the application site and scene.

The fifth purpose of the present invention is to provide a cartilage repair matrix, including polymers and/or their derivative, and a above-mentioned recombinant collagen protein.

Further, the stated polymer includes natural polymers and synthetic polymers; further, the stated natural polymer is selected at least from any one of hyaluronic acid, chitosan, alginic acid, and cellulose; further, the stated synthetic polymer is selected at least from any one of PLA, PGA, PLCL, and PVA.

The sixth purpose of the present invention is to provide the above-mentioned preparation method of a cartilage repair matrix, comprising the following steps:
(1) Dissolve the polymer derivative in a buffer solution, added with EDC/sNHS solution for reaction;
(2) Add the recombinant collagen protein stated in any one of claims 1 to 7 to the reaction system of step (1) for reaction, and dialyze and dry the solution obtained after reaction to obtain the composite matrix;
(3) Dissolve the composite matrix, added with a photoinitiator solution to obtain a cartilage repair matrix;

Humanized collagen cannot meet the requirements of cartilage tissue engineering due to its small molecular weight, poor mechanical properties, and difficulty in forming glue even after modification. In the example of the present invention, hyaluronic acid is first moderately chemically modified with methacrylic anhydride, introduced with unsaturated carbon-carbon double bonds, then added with EDC/sNHS to activate the carboxyl group in hyaluronic acid, then added with recombinant humanized collagen so that the amino group of the collagen and the activated carboxyl group of the hyaluronic acid are amidated to make the two coupled. Finally, the composite hydrogel obtained through photo-crosslinking is of good mechanical properties and biocompatibility, meanwhile the photo-crosslinking method makes the operation easier, which can meet the requirements of cartilage tissue engineering, and effectively address the clinical requirements for irregular cartilage defect repair and filling.

The EDC described in the present invention refers to 1-(3-dimethylaminopropyl)-3-ethylcarbodiimide hydrochloride, and NHS refers to N-hydroxysuccinimide.

Further, the polymer derivative described in step (1) is hyaluronic acid modified using methacrylic anhydride;
Further, the molecular weight of the modified hyaluronic acid is within 100 to 1500 kDa, preferably 300 to 600 kDa, more preferably 300 kDa;
Further, the modified grafting degree of hyaluronic acid described in step (1) is within 20 to 60%, preferably 30 to 50%;
Further, in step (1), after the addition of EDC and sNHS, the concentration of sNHS is within 0.5 to 5 mM and the concentration of EDC is within 0.5 to 5 mM; further, the concentration of sNHS is within 1 to 3 mM and the concentration of EDC is within 2 to 4 mM.

In the present invention, in step (1), the pH of the mixed solution after the addition of the EDC/sNHS solution is within 4 to 5, preferably 4.75;
The pH of the mixed solution after reaction is within 7 to 8, and the reaction time is within 2 to 5h; preferably, the pH is 7.4, and the reaction time is 3h.

In the present invention, in step (2), the mass ratio of recombinant collagen protein: polymer derivative is 0.5-30: 2-10; further 0.5-20: 6; further 1-11: 6;
Further, the reaction time in step (2) is 5 to 15h, preferably 8 to 12h;
Further, in step (2), the reacted solution is loaded into a dialysis bag with a molecular weight cut-off of 8,000 to 14,000, dialyzed with deionized water for 2 to 4 days, lyophilized to give the composite matrix.

The photoinitiator used in the present invention is at least one of LAP, 2-hydroxy-4'-(2-hydroxyethoxy)-2-methylpropiophenone (I2959), with a final concentration of 0.01 - 0.05% by mass volume, preferably 0.05%.

In the present invention, the concentration of the composite matrix after the addition of the photoinitiator in step (3) is 2 - 30 mg/ml, further 5 - 20 mg/ml, and further 7 - 17 mg/ml.

The cartilage repair matrix described in the present invention is a product including the stated recombinant collagen protein and can be used as a component of medical devices including, but not limited to, implantable materials, tissue engineering scaffold, soft tissue filler materials, cellular or other active substance vector materials.

The cartilage repair matrix described in the present invention can be prepared as a cartilage repair hydrogel with the addition of cells, or applied directly to the site of cartilage defects without the addition.

The seventh purpose of the present invention is to provide a preparation method of a cartilage repair hydrogel, specifically, the cartilage repair matrix prepared by the stated method is compounded with chondrocytes or bone marrow mesenchymal stem cells, then injected into the cartilage injury site, and then exposed to light to form a cartilage repair hydrogel.

The wavelength of the stated light is adaptively selected according to the initiator used.

The cartilage repair hydrogel described in the present invention also comprises physiologically acceptable vector materials.

The vector materials herein include, but are not limited to, water-soluble vector materials (e.g., polyethylene glycol, polyvinylpyrrolidone, and organic acids), insoluble vector materials (e.g., ethyl cellulose and cholesteryl stearate), and enteric-soluble vector materials (e.g., cellulose acetate phthalate and carboxymethyl hydroxyethyl cellulose).

The stated composition can also be used in combination with other functional materials as desired, such as compositions with antibacterial and antimicrobial effects, anti-aging compositions, anticoagulant compositions, antioxidant compositions, and growth factors.

The stated compositions can be prepared according to conventional techniques for preparations or cosmetics, such as mixing the recombinant collagen protein of the present invention as an active ingredient with vectors to make the desired dosage form according to conventional techniques. The compositions claimed by the present invention can be formulated into a variety of dosage forms as desired, including but not limited to oral preparations, mucosal administration preparations, injection preparations, inhalation preparations and topical preparations.

The products described in the present invention include, but are not limited to, drugs, food, health care products or cosmetics, daily necessities.

In this specification, the word of "can" includes the meaning of performing certain processing and the meaning of not performing certain processing.

In practical applications, the recombinant collagen of the present invention and the stated composition can be directly administered to patients as medicines, or mixed with appropriate vectors or excipients and then administered to patients. The vector materials in the present invention include, but are not limited to, water-soluble vector materials (e.g., polyethylene glycol, polyvinylpyrrolidone, and organic acids), insoluble vector materials (e.g., ethyl cellulose and cholesteryl stearate), and enteric-soluble vector materials (e.g., cellulose acetate phthalate and carboxymethyl hydroxyethyl cellulose). Preferred among these are water-soluble vector materials. A variety of dosage forms can be made using these materials, including, but not limited to, tablets, capsules, drops, aerosols, pills, powders, solutions, suspensions, emulsions, granules, liposomes, transdermal agents, buccal tablets, suppositories, and lyophilized powder injections. Among them, the suppositories can be as vaginal suppositories, or vaginal rings, or ointments, creams or gels suitable for vaginal application.

The stated dosage form can be a conventional dosage form, a sustained release preparation, a controlled release preparation and various particulate delivery systems. To formulate unit dosage forms such as tablets, a wide range of well-known vectors in the field can be extensively utilized. Examples with respect to the vectors are: diluents and absorbents, such as starch, dextrin, calcium sulfate, lactose, mannitol, sucrose, sodium chloride, glucose, urea, calcium carbonate, kaolin, microcrystalline cellulose and aluminum silicate; wetting agents and binders such as water, glycerin, polyethylene glycol, ethanol, propanol, starch syrup, dextrin, molasses, honey, glucose solution, mucilago gummi arabici, gelatin syrup, carboxymethyl cellulose sodium, gum lac, methyl cellulose, potassium phosphate, and polyvinylpyrrolidone; disintegration agents, such as dried starch, alginate, agar powder, laminaran, sodium bicarbonate and citrate, calcium carbonate, polyoxyethylene, sorbitan fatty acid esters, sodium dodecyl sulfate, methyl cellulose, and ethyl cellulose; disintegration inhibitors, such as sucrose, glyceryl tristearate, cocoa butter, and hydrogenated oils; absorption enhancers , such as quaternary ammonium salts and sodium dodecyl sulfate; lubricants, such as talc, silica, corn starch, stearate, boric acid, liquid paraffin, and polyethylene glycol. The tablets may further be made into coated tablets, such as sugar-coated tablets, film-coated tablets, enteric-coated tablets, or double-layer tablets and multi-layer tablets. In order to make the unit-delivery dosage form into pills, a wide range of vectors known in the field can be used. Examples with respect to vectors are: diluents and absorbents, such as glucose, lactose, starch, cocoa butter, hydrogenated vegetable oils, polyvinylpyrrolidone, Gelucire, kaolin, and talc; binders such as gum arabic, yarrow, gelatine, ethanol, honey, liquid sugar, rice paste and batter; disintegrating agents, such as agar powder, dried starch, alginate, sodium dodecylsulphonate, methyl cellulose, and ethyl cellulose. In order to make a unit-delivery dosage form into suppositories, a wide variety of vectors known in the field can be used. Examples of the vectors are: polyethylene glycol, lecithin, cocoa butter, higher alcohols, esters of higher alcohols, gelatin, and semi-synthetic glycerides. In order to make unit-delivery dosage forms into preparations for injection such as solutions, emulsions, lyophilized powders and suspensions, all diluents commonly used in the art can be used, such as water, ethanol, polyethylene glycol, 1,3-propylene glycol, ethoxylated isostearyl alcohols, multi-oxidized isostearyl alcohols, and polyoxyethylene sorbitol fatty acid ester. Additionally, in order to prepare isotonic injection solution, an appropriate amount of sodium chloride, glucose or glycerol may be added to the preparation for injection, in addition to conventional cosolvents, buffers, and pH adjusters. In addition, if desired, coloring agents, preservatives, perfumes, corrigens, sweeteners, or other materials can also be added to the pharmaceutical preparation.

Using the above dosage forms can be administered via injection, including subcutaneous, intravenous, intramuscular and intraperitoneal injections, and intracisternal injection or infusion; cavity administration, such as transrectal, vaginal and sublingual administration; respiratory administration, such as transnasal administration; mucosal administration.

Preferred among the above routes of administration is injective administration, and the preferred route of injection is subcutaneous injection.

The administration dosage of the recombinant collagen of the present invention and the compositions described above depends on many factors, such as the nature and severity of the disease to be prevented or treated, the gender, age, body weight and individual response of the patient or animal, the specific active ingredient to be used, the route and frequency of administration. The above dosages may be administered in the form of a single dose or divided into several dose, e.g., two, three or four doses. For any particular patient, the specific effective dose shall be based on a variety of factors, the stated factors including: the disorder being treated and the severity of the disorder; the activity of the specific active ingredient employed; the specific combination employed; the age, body weight, general health, gender, and diet; the time of administration, route of administration, and excretion rate of the specific active ingredient employed; the duration of the treatment; the medication used in combination with, or concurrently with, the specific active ingredient employed; and similar factors known in the medical field.

For example, it is common practice in the art to start dosages of active ingredients below the level required to achieve the desired therapeutic effect and gradually increase the dosage until the desired effect is obtained.

Beneficial effects:
(1) The recombinant collagen protein claimed by the present invention exhibits significant integrin binding activity, promoting cell adhesion, proliferation, differentiation, and repairing cartilage tissue defects, among other effects. It has promising prospects for application.
(2) The present invention covalently cross-links hyaluronic acid and humanized collagen to form a composite hydrogel matrix with stable morphology which has the following characteristics: good mechanical properties, which can well maintain the morphology of the complex; good biocompatibility. Additionally, the photo-crosslinking makes the operation easier, which can meet the requirements of cartilage tissue engineering, and effectively address the clinical requirements for irregular cartilage defect repair and filling.

### Figure Legend

Fig. 1 The results of cell adhesion growth of fibroblasts.
Fig. 2 The OD values of cell adhesion of fibroblasts;
Fig. 3 The results of cell adhesion growth of chondrocytes;
Fig. 4 The OD values of cell adhesion of chondrocytes;
Fig. 5 The results of adhesion experiments on human gingival fibroblasts with different concentrations of sequence A and sequence B;
Fig. 6 The morphology of chondrocyte/hydrogel complexes after 1, 7, 14 and 21 days of in vitro culture;
Fig.7 The OD values of chondrocyte/hydrogel complexes for chondrocyte adhesion after 1, 7, and 14 days of in vitro culture;
Fig. 8 CCK-8 plots of chondrocyte/hydrogel complexes for chondrocytes after 1, 7, and 14 days of in vitro culture;
Fig.9 The staining results of chondrocyte/hydrogel complexes;
Fig.10 The results of immunohistochemical staining of type II collagen of chondrocyte/hydrogel complexes;
Fig.11 The GAG quantitative detection results of chondrocyte/hydrogel complex.

### Specific embodiments

The present invention is described in detail below in combination with the accompanying drawings and specific embodiments. This embodiment is implemented on the premise of the technical solution of the present invention, and detailed implementation mode and specific operation process are given, but the protection scope of the present invention is not limited to the following embodiments.

Throughout the patent specification, unless otherwise specified, terms used in the present invention should be understood to have the meanings as commonly used in the art. Accordingly, unless otherwise defined, all technical and scientific terms used in the present invention have the same meaning as generally understood by those skilled in the art to which the present invention belongs. In the event of a conflict, this specification takes precedence.

### Embodiment 1

Encode a nucleotide sequence of the stated recombinant collagen protein, construct vectors comprising the nucleotide sequence, construct and screen out the host cells (which can be the prokaryotic cells or the eukaryotic cell) comprising the vectors, culture the host cells for the expression of the protein under appropriate conditions, and collect and purify the expressed recombinant collagen protein.

The term "vector" described in the present invention refers to a nucleic acid delivery tool that can insert the polynucleotide into the host cells. When the vector enables expression of the protein encoded by the inserted polynucleotide, the vector is called an expression vector. A vector can be introduced into a host cell by transformation, transduction or transfection so that the genetic material element it carries can be expressed in the host cells. The vectors are well known to those skilled in the art and include, but are not limited to: plasmids; phagemids; Coase plasmids; artificial chromosomes such as yeast artificial chromosomes (YAC), bacterial artificial chromosomes (BAC), or artificial chromosomes of P1 origin (PAC); phages such as λ phage, M13 phage and animal viruses. The vectors may contain a variety of elements for controlling expression including, but not limited to, promoter sequences, transcription start sequences, enhancer sequences, selection elements, and reporter genes. Alternatively, the vectors can contain a replication origin. The vectors can contain the nucleic acid of the present invention for introduction into cells for expression. The vectors can contain expression control elements linked to the stated nucleic acids, such as promoters, terminators and/or enhancers.

Recombination and transcription of genes involved in the present invention refers to synthesizing nucleotide sequences encoding recombinant collagen, cloning the nucleotide sequences into expression vectors by conventional techniques, then transforming the expression vectors into host cells, finally constructing and screening to obtain engineered strains. Transformation methods include, but are not limited to, electroporation and CaCl₂ transformation; the expression vectors can be commonly used vectors such as pET26, pET32, pGEX-6p, pPIC9, and pPIC9K, also can be plasmid, phages, virus, or other vectors.

The term "host cell" described in the present invention refers to a cell into which nucleic acid molecules have been introduced by molecular biology techniques. These techniques include transfection with viral vectors, transformation with plasmid vectors, and introduction of naked DNA by electroporation, lipofection, and particle gun. The host cells can be eukaryotic or prokaryotic cells: microbial cells such as Enterobacteriaceae cells (e.g., E. coli BL21 and DH5α); eukaryotic cells such as yeast (e.g., pichia pastoris and saccharomyces cerevisiae) and CHO cells.

The fermentation culture involved in the present invention refers to the induced fermentation culture of an engineered bacterial strain screened and certified for high protein expression with a suitable fermentation culture medium under suitable temperature, pressure and pH conditions in a sterile fermentation tank.

The separation and purification of proteins involved in the present invention refers to that the bacteria liquid is obtained after lysis, homogenization, separation and other treatments, then the recombinant collagen protein is obtained through conventional protein separation and purification techniques. The separation and purification techniques include, but are not limited to, filtration, centrifugation, salting out, dialysis, liquid chromatography, ion exchange chromatography, and affinity chromatography.

In the embodiment of the present invention, taking E. coli as the host cell as an example, the gene recombination and transcription are specifically as follows:
1. Preparation of recombinant collagen protein with fermentation of E. coli

Gene recombination and transcription: Use PCR method for codon optimization and splicing recombination of DNA fragments, construct the pET-32a and then transfer it into the E. coli expression strain BL21, after cultivation and screen, obtain the genetically engineered Escherichia coli with high protein expression.

Fermentation culture: Pick single colony of the genetically engineered Escherichia coli from LB plate, put it in 100mL triangular flask containing 10mL LB medium, and incubate it at 37°C, 220rpm for 12 - 16h; inoculate the bacterial solution, at the ratio of 1:100, into a fermentation tank containing LB medium to amplify the culture, and incubate the culture at 37°C, 220rpm for 3h until the OD600 is about 0.6, then add 0.5 mM IPTG, and collect the bacteria by centrifugation after 20h of induction culture at 16°C.

Separation and purification of protein: Re-select the bacteria with Tris buffer, dissolve the bacteria completely by high-speed stirring, and collect the supernatant by centrifugation and cool the supernatant down to 4 °C; filter the supernatant with 1 µm, 0.45 µm and 0.22 µm membranes sequentially, purify with affinity chromatography to obtain recombinant collagen protein.

### 2. Cell culture method

1) Sample preparation: Prepare the recombinant collagen protein with PBS to a certain concentration solution (such as 0.5 mg/ml), dilute the animal-derived collagen solution with PBS to the same concentration (such as 0.5 mg/ml), and PBS is the blank control group;
2) Plate spreading: Add the experimental solutions into 96-well plates, 100 µL per well, 5 wells in each group, and let the plates stand overnight at 4 °C;
3) Closure: After the plate spreading, remove the liquid from the well plates and wash it twice with 200 µL of PBS solution, add 100 µL of heat-inactivated 1% BSA-PBS solution, and incubate for 1 h at 37 °C in an incubator with 5% CO₂.
4) Cell inoculation: After the incubation, remove the liquid from the well plates and wash it twice with 200 µL of PBS solution, add 100 µL of cell suspension with a density of 5x10⁴ - 1×10⁶ cells/ml to each well, and incubate for 1 h at 37°C in an incubator with 5% CO₂;
5) Detection: After the incubation, remove the liquid from the well plates and wash it twice with 200 µL of PBS solution, add 150 µL of CCK-8, and incubate it for 1 h at 37°C in an incubator with 5% CO₂, and then add 100 µL of the test solution to a new 96-well plate to detect the absorbance value (OD) at 450 nm.

### Embodiment 1:

The following three types of recombinant collagen protein containing different amino acid sequences were prepared using the method of Embodiment 1.

A series of experiments such as the enhancement of cell adhesion, migration, and functional expression were performed using the recombinant collagen protein obtained by directly repeating the repetitive unit with the above-mentioned amino acid sequence for 16 times. The sequence A was repeatedly connected 16 times, sequence A: GER GAP GFR GPA GPN GIP GEK GPA GER GAP, the whole sequence is as follows (SEQID NO. 1):
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP.

Another type of recombinant collagen protein obtained by directly repeating the repetitive units with different above-mentioned characteristic amino acid sequences 4 times has different effects on cell adhesion and proliferation. The sequence B was repeatedly connected 4 times, sequence B: GEK GSP GAD GPA GAP GTP GPQ GIA GQR GVV GLP GQR GER GFP GLP GPS GEP GKQ GPS GAS, the whole sequence is as follows (SEQID NO.2):
GEKGSPGADG PAGAPGTPGP QGIAGQRGVV
GLPGQRGERG FPGLPGPSGE PGKQGPSGAS
GEKGSPGADG PAGAPGTPGP QGIAGQRGVV
GLPGQRGERG FPGLPGPSGE PGKQGPSGAS
GEKGSPGADG PAGAPGTPGP QGIAGQRGVV
GLPGQRGERG FPGLPGPSGE PGKQGPSGAS
GEKGSPGADG PAGAPGTPGP QGIAGQRGVV
GLPGQRGERG FPGLPGPSGE PGKQGPSGAS.

The recombinant collagen protein obtained by repeated connection of the above-mentioned repetitive unit for 16 times has a significantly reduced adhesion and proliferation effect on cells after they were added with tags. The sequence C was repeatedly connected 16 times, sequence C: HHHHHH GER GAP GFR GPA GPN GIP GEK GPA GER GAP, the whole sequence is as follows (SEQID NO.3):
HHHHHHGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAPGERG APGFRGPAGP NGIPGEKGPA
GERGAP.

Fibroblast adhesion, chondrocyte adhesion experiments, and human gingival fibroblast pro-adhesion experiments were performed on the above three collagens using the cell culture method in Embodiment 1.

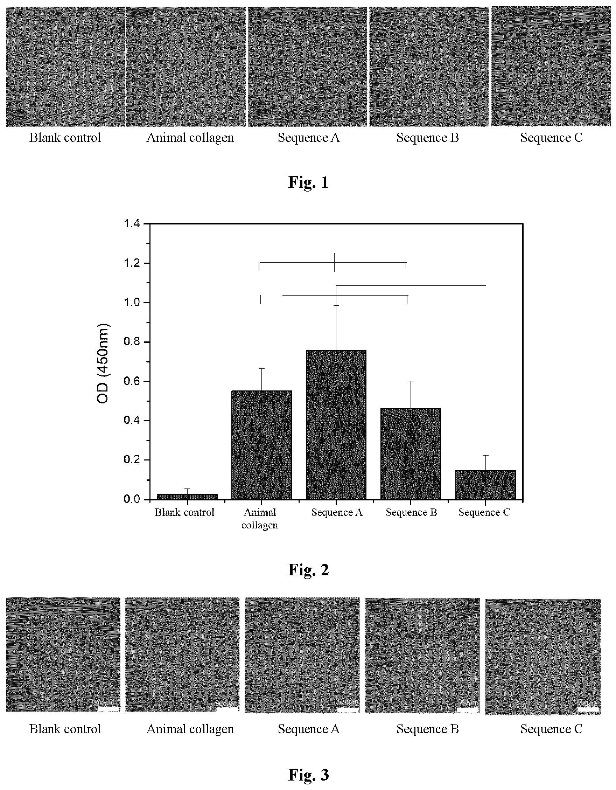

The experimental results are shown in Figures 1 - 3.

The results in Fig. 1 showed that the recombinant collagen group obtained by repeated connection of sequence A with many characteristic amino acid sequences has obvious fibroblast adhesion effect and good cell morphology; the recombinant collagen group obtained by repeated connection of sequence B with reduced characteristic amino acid sequences has similar fibroblast adhesion effect and cell morphology as the animal collagen group; the adhesion of fibroblasts in the recombinant collagen group obtained by repeated connection of the tagged sequence C was significantly reduced, and the adhesion of fibroblasts in the blank control group was also very little.

The results in Fig. 2 showed that the recombinant collagen obtained by repeated connection of sequence A with many characteristic amino acid sequences can promote the adhesion of fibroblasts, followed by animal collagen, then the recombinant collagen obtained by repeated connection of sequence B; the adhesion amount of fibroblasts in the three groups was significantly higher than that of the recombinant collagen obtained by repeated connection of sequence C and the blank control; the adhesion amount of fibroblasts in the recombinant collagen group of sequence C was slightly higher than that of the blank control.

The results in Fig. 3 showed that the adhesion of chondrocytes in the animal collagen group was the most obvious, almost covering the whole field of view; the sequence A recombinant collagen group followed, with obvious and relatively concentrated adhesion of chondrocytes and a good cell morphology; the chondrocytes adhered to the sequence B recombinant collagen group were more dispersed, with a small part of them concentrated, and the cell morphology was good; the chondrocytes adhered to the sequence C recombinant collagen group were dispersed and the number of chondrocytes was low; in the blank control group, only a small number of chondrocytes were adhered and scattered.

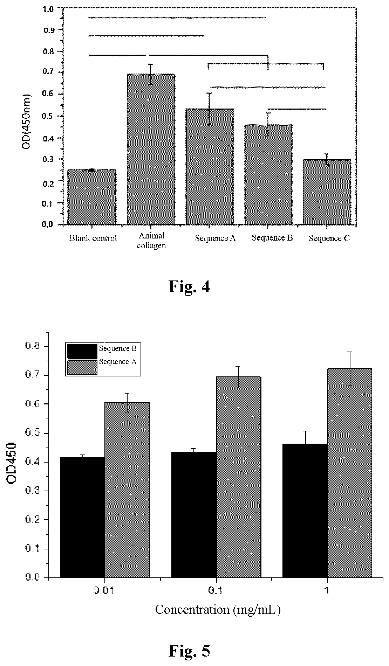

The results in Fig. 4 showed that in the promotion of chondrocyte adhesion, the animal collagen is most conducive to the promotion of chondrocyte adhesion, which is significantly higher than the other groups; the sequence A recombinant collagen group, which has more characteristic amino acid sequences, is significantly higher than the sequence B recombinant collagen group, the sequence C recombinant collagen group and the blank control group; the sequence B recombinant collagen group is significantly higher than the sequence C recombinant collagen group and the blank control group; the sequence C recombinant collagen group is slightly higher than the blank control group, but there was no statistically significant difference between the two groups.

The results in Fig. 5 showed that according to the results of the adhesion promotion experiments on human gingival fibroblasts, the sequence A recombinant collagen group, which has more characteristic amino acid sequences, was more conducive to the adhesion of human gingival fibroblasts than the sequence B recombinant collagen group at any concentration stated; as the concentration of the recombinant collagen increased, the adhesion of human gingival fibroblasts also gradually increased.

### Embodiment 2 Application of recombinant collagen protein in promoting cartilage repair

The method of Embodiment 1 was used to prepare recombinant collagen protein that conformed to the amino acid sequence characteristics stated in the present invention. The core sequence of the amino acid sequence of the recombinant humanized collagen is preferably GERGAPGFRGPAGPNGIPGEKGPAGERGAP (sequence A), which is connected repeatedly for 16 times and used to promote cartilage repair. The specific steps were as follows:
(1) Methacrylated hyaluronic acid (HA-MA) with a molecular weight of 300 KDa and a degree of modification of 37% was dissolved in MES buffer solution to obtain a 1 mM solution of methacrylated hyaluronic acid;
(2) sNHS and EDC both in solid form were added sequentially to the mixed solution so that the concentration of sNHS was 2mM and the EDC concentration was 3mM, and a 1.0 M NaOH solution or a 1.0 M HCl solution was used to maintain the pH of the mixed solution at 4.75;
(3) The solution was stirred at room temperature for 2 h;
(4) The pH of the reaction system was adjusted to 7.4 with 1.0 M NaOH solution;
(5) The recombinant humanized collagen was added to the reaction system so that the mass ratio of recombinant humanized collagen and methacrylated hyaluronic acid in the mixed solution was 1:6, and the reaction was stirred for 8 h;
(6) The resulting solution the reaction was loaded into a dialysis bag with a molecular weight cut-off of 8,000-14,000, and after three days of dialysis with deionized water, it was lyophilized with a freeze dryer to obtain the lyophilized sponge HA-rhCol III;
(7) The lyophilized sponge in step (6) was dissolved in 0.01 M PBS, added with LAP photoinitiator solution (the final mass to volume ratio of 0.05%), so that the final concentration of HA-rhCol III solution was 7 mg/ml;
(8) The precursor solution in step (7) was placed in a silicone mold with a size of Φ6mm×2.5mm, illuminated under ultraviolet light for 1 minute to obtain a hyaluronic acid hydrogel containing recombinant humanized collagen (HA-rhCol III).

### Embodiment 3 Application of recombinant collagen protein in promoting cartilage repair

The method of Embodiment 1 was used to prepare recombinant collagen protein that conformed to the amino acid sequence characteristics stated in the present invention. The core sequence of the amino acid sequence of the recombinant humanized collagen is preferably GERGAPGFRGPAGPNGIPGEKGPAGERGAP (sequence A), which is connected repeatedly for 16 times and used to promote cartilage repair. The specific steps were as follows:
(1) Methacrylated hyaluronic acid HA-MA with a molecular weight of 100 KDa and a degree of modification of 50% was dissolved in MES buffer solution to obtain a 2 mM methacrylated hyaluronic acid solution;
(2) sNHS and EDC both in solid form were added sequentially to the mixed solution so that the concentration of sNHS was 3mM and the EDC concentration was 5mM, and a 0.5 M NaOH solution or a 0.5 M HCl solution was used to maintain the pH of the mixed solution at 4.75;
(3) The solution was stirred at room temperature for 2 h;
(4) The pH of the reaction system was adjusted to 7.4 with 1.0 M NaOH solution;
(5) The recombinant humanized collagen was added to the reaction system so that the mass ratio of recombinant humanized collagen and methacrylated hyaluronic acid in the mixed solution was 11:6, and the reaction was stirred for 12 h;
(6) The resulting solution the reaction was loaded into a dialysis bag with a molecular weight cut-off of 8,000-14,000, and after three days of dialysis with deionized water, it was lyophilized with a freeze dryer to obtain the lyophilized sponge HA-rhCol III;
(7) The lyophilized sponge in step (6) was dissolved in 0.01 M PBS, added with LAP photoinitiator solution (the final mass to volume ratio of 0.01%), so that the final concentration of HA-rhCol III solution was 17 mg/ml;
(8) The precursor solution in step (7) was placed in a silicone mold with a size of Φ6mm×2.5mm, illuminated under ultraviolet light for 1 minute to obtain HA-rhCol III hydrogel.

### Embodiment 4 Application of recombinant collagen protein in promoting cartilage repair

The method of Embodiment 1 was used to prepare recombinant collagen protein that conformed to the amino acid sequence characteristics stated in the present invention. The core sequence of the amino acid sequence of the recombinant humanized collagen is preferably GERGAPGFRGPAGPNGIPGEKGPAGERGAP (sequence A), which is connected repeatedly for 16 times (SEQID NO.1) and used to promote cartilage repair. The specific steps were as follows:
(1) Methacrylated hyaluronic acid HA-MA with a molecular weight of 600 KDa and a degree of modification of 30% was dissolved in MES buffer solution to obtain a 1 mM methacrylated hyaluronic acid solution;
(2) sNHS and EDC both in solid form were added sequentially to the mixed solution so that the concentration of sNHS was 1mM and the EDC concentration was 3mM, and a 1.0 M NaOH solution or a 1.0 M HCl solution was used to maintain the pH of the mixed solution at 4.75;
(3) The solution was stirred at room temperature for 3 h;
(4) The pH of the reaction system was adjusted to 7.4 with 1.0 M NaOH solution;
(5) The recombinant humanized collagen was added to the reaction system so that the mass ratio of recombinant humanized collagen and methacrylated hyaluronic acid in the mixed solution was 0.5:6, and the reaction was stirred for 8 h;
(6) The resulting solution the reaction was loaded into a dialysis bag with a molecular weight cut-off of 8,000 - 14,000, and after three days of dialysis with deionized water, it was lyophilized with a freeze dryer to obtain the lyophilized sponge HA-rhCol III;
(7) The lyophilized sponge in step (6) was dissolved in 0.01 M PBS, added with LAP photoinitiator solution (the final mass to volume ratio of 0.05%), so that the final concentration of HA-rhCol III solution was 17 mg/ml;
(8) The precursor solution in step (7) was placed in a silicone mold with a size of Φ6mm×2.5mm, illuminated under ultraviolet light for 2 minute to obtain HA-rhCol III hydrogel.

### Embodiment 5 Application of recombinant collagen protein in promoting cartilage repair

The method of Embodiment 1 was used to prepare recombinant collagen protein that conformed to the amino acid sequence characteristics stated in the present invention. The core sequence of the amino acid sequence of the recombinant humanized collagen is preferably GERGAPGFRGPAGPNGIPGEKGPAGERGAP (sequence A), which is connected repeatedly for 16 times (SEQID NO.1) and used to promote cartilage repair. The specific steps were as follows:
(1) Methacrylated hyaluronic acid HA-MA with a molecular weight of 1,000 KDa and a degree of modification of 40% was dissolved in MES buffer solution to obtain a 0.6 mM methacrylated hyaluronic acid solution;
(2) sNHS and EDC were added sequentially to the mixed solution so that the concentration of sNHS was 5mM and the EDC concentration was 5mM, and a 0.2 M NaOH solution or a 0.2 M HCl solution was used to maintain the pH of the mixed solution at 4.75;
(3) The solution was stirred at room temperature for 3 h;
(4) The pH of the reaction system was adjusted to 7.4 with 0.5 M NaOH solution;
(5) The recombinant humanized collagen was added to the reaction system so that the mass ratio of recombinant humanized collagen and methacrylated hyaluronic acid in the mixed solution was 11:6, and the reaction was stirred for 12 h;
(6) The reacted solution was loaded into a dialysis bag with a molecular weight cut-off of 8,000 - 14,000, and after three days of dialysis with deionized water, it was lyophilized with a freeze-dryer to obtain the product HA-rhCol III;
(7) The lyophilized sponge in step (6) was dissolved in 0.01 M PBS, added with I2959 photoinitiator solution (the final mass to volume ratio of 0.03%), so that the final concentration of HA-rhCol III solution was 17 mg/ml;
(8) The precursor solution in step (7) was placed in a silicone mold with a size of Φ6mm×2.5mm, illuminated under ultraviolet light for 1 minute to obtain HA-rhCol III hydrogel.

### Embodiment 2:

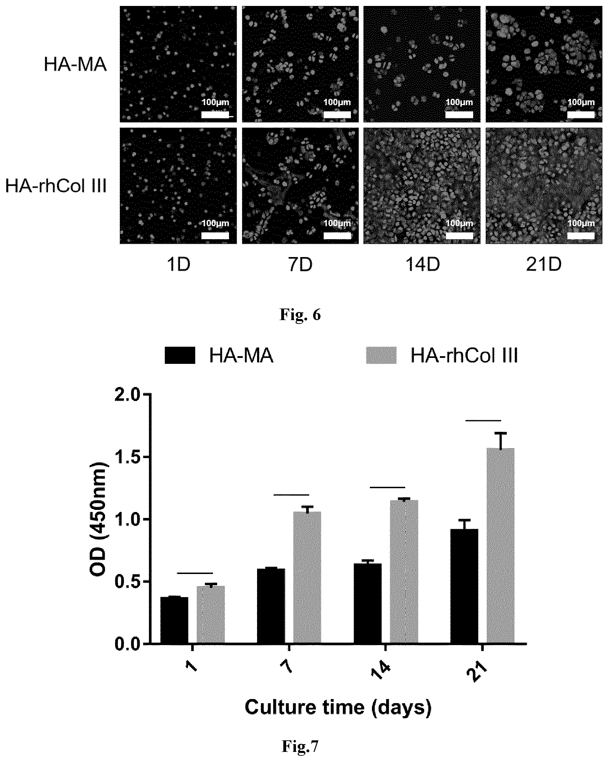

The recombinant humanized collagen-modified hyaluronic acid hydrogel prepared in Embodiment 2 has the following results in cell experiments:
The results of Fig. 6 and 7 showed that chondrocytes in HA-MA hydrogel were cultured up to 21 days and chondrocytes aggregated in situ to grow in clusters due to the lack of adhesion sites. Whereas, chondrocytes in HA-rhCol III hydrogels showed partial spreading when cultured up to 7 days, and at 14 and 21 days, the spreading growth phenomenon of cells was more obvious and evenly distributed. This indicated that rhCol III was favorable for cell adhesion, migration and proliferation, and HA-rhCol III hydrogel can promote the proliferation of chondrocytes better than HA-MA hydrogel.

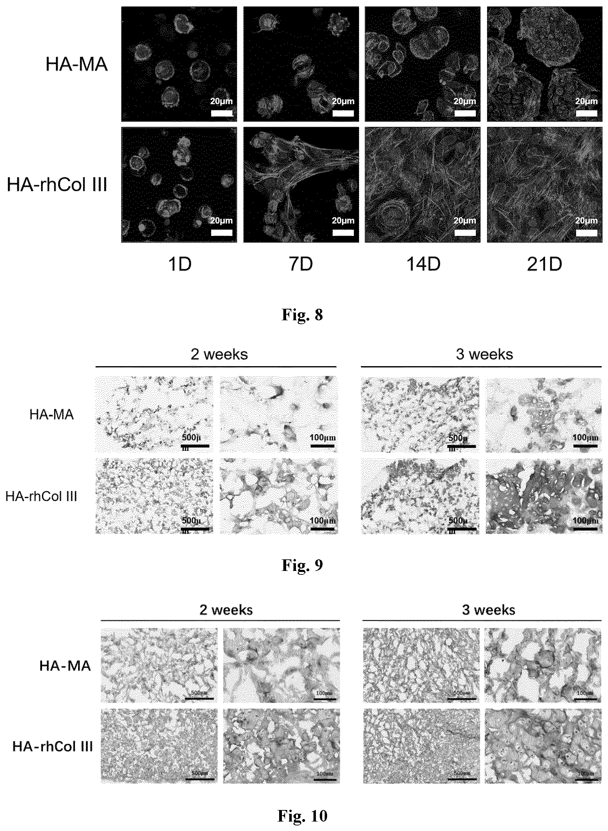

The results of Fig. 8 showed that the cell nucleus in the hydrogel of HA-rhCol III group did not show any deformation or shrinkage and the actin of the cells in the hydrogel of the two groups was in the fusiform shape when the cells were cultured for 7 and 14 days, indicating that the cells were spread well inside the gel; whereas, the cells of the HA-MA group proliferated in situ in the form of cell clusters at all time points. The results suggested that rhCol III provided a suitable site for cell adhesion, which allowed chondrocytes to adhere faster and provided conditions for cell proliferation.

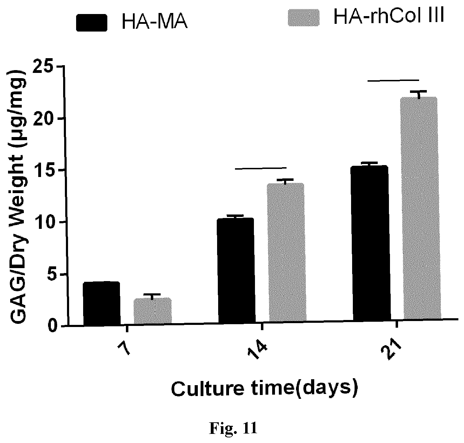

Histological and immunohistochemical staining of type II collagen (Fig. 9 and 10) showed that there were more obvious polysaccharide and protein staining areas in the HA-rhCol III gels, suggesting that the addition of rhCol III could promote the functional expression of chondrocytes. The results of GAG quantification (Fig. 11) further confirmed that the addition of rhCol III was more conducive to the secretion of chondrocyte extracellular matrix.

The above description of the examples is intended to facilitate the understanding and use of the invention by those of ordinary skill in the art. Persons skilled in the art can obviously easily make various modifications to these examples and apply the general principles illustrated in the present invention to other examples without creative effort. Therefore, the present invention is not limited to the above examples, and improvements and modifications made by those skilled in the art in accordance with the disclosure of the present invention but within the scope of the present invention should also fall within the scope of protection of the present invention.

## Claims

1. A recombinant collagen protein **characterized in that** the amino acid sequence of the stated recombinant collagen protein comprises N basic repetitive units and the stated basic repetitive unit comprises n1 amino acid sequences with the following characteristic: "G-Xaa₁-Xaa₂-G-E-Xaa₃"; the 3' end and the 5' end of the basic repetitive unit are connected to form the amino acid sequence with the above characteristic;
Wherein, N is an integer of 4 or more; n1 is an integer of 3 or more.

2. The recombinant collagen protein according to claim 1, **characterized in that** N is an integer within 4 to 300.

3. The recombinant collagen protein according to claim 1, **characterized in that** N is an integer within 4 to 200.

4. The recombinant collagen protein according to claim 1, **characterized in that** the characteristic amino acid sequences are arranged consecutively or at intervals in the basic repetitive units.

5. The recombinant collagen protein according to claim 1, **characterized in that** the amino acid sequence of the stated recombinant collagen protein have the following characteristics: 
[-G-E-Xaa3-Yaa1-(G-Xaa1-Xaa2-G-E-Xaa3)n2-Yaa2-(G-Xaa1-Xaa2-G-E-Xaa3)n3-Yaa3-
(G-Xaa1-Xaa2-G-E-Xaa3)n3-Yaa4-(G-Xaa1-Xaa2-G-E-Xaa3)n4-…-Yaan-(G-Xaa1-Xaa2-
G-E-Xaa3)n-Yaan+1-G-Xaa1-Xaa2-]N
Wherein, the stated Xaa₁ is a non-polar hydrophobic amino acid; the stated Xaa₂ is one of serine (S), alanine (A), proline (P), and oxyproline (O); the stated Xaa₃ is a basic amino acid;
Each occurrence of Yaa₁, Yaa₂, Yaa₃, Yaa₄, ..., Yaaₙ, Yaaₙ₊₁ is independently selected from none, one or more different or identical amino acids;
n2, n3, n4, ..., n all are an integer above 0, but they are not 0 at the same time.

6. The recombinant collagen protein as claimed in claim 1, **characterized in that** the stated recombinant collagen protein sequence does not contain a protein tag.

7. The recombinant collagen protein as claimed in claim 1, **characterized in that** the stated recombinant collagen protein has the amino acid sequence as shown in SEQID NO. 1.

8. The preparation method of recombinant collagen protein as claimed in claim 1, **characterized in that** the recombinant collagen protein is obtained through host cell expression, extraction and purification using gene recombination technology; the amino acid sequence of the stated recombinant collagen protein is obtained by repeated connection of basic repetitive units with multiple repetitions.

9. The preparation method of recombinant collagen protein as claimed in claim 8, **characterized in that** the amino acid sequence of the stated recombinant collagen protein comprises N basic repetitive units and the stated basic repetitive unit comprises n1 amino acid sequences with the following characteristic: "G-Xaa₁-Xaa₂-G-E-Xaa₃"; the 3' end and the 5' end of the basic repetitive unit are connected to form the amino acid sequence with the above characteristic;
Wherein, N is an integer greater than 4; n1 is an integer greater than 3.

10. The preparation method of recombinant collagen protein as claimed in claim 9, **characterized in that characterized in that** the characteristic amino acid sequences are arranged consecutively or at intervals in the basic repetitive units.

11. The preparation method of recombinant collagen protein as claimed in claim 8, **characterized in that** the stated recombinant collagen protein sequence does not contain protein tags;
The amino acid sequence of the stated recombinant collagen protein exhibits the following characteristics: 
[-G-E-Xaa3-Yaa1-(G-Xaa1-Xaa2-G-E-Xaa3)n2-Yaa2-(G-Xaa1-Xaa2-G-E-Xaa3)n3-Yaa3-
(G-Xaa1-Xaa2-G-E-Xaa3)n3-Yaa4-(G-Xaa1-Xaa2-G-E-Xaa3)n4-…-Yaan-(G-Xaa1-Xaa2-
G-E-Xaa3)n-Yaan+1-G-Xaa1-Xaa2-]N
Wherein, the stated Xaa₁ is a non-polar hydrophobic amino acid; the stated Xaa₂ is one of serine (S), alanine (A), proline (P), oxyproline (O); and the stated Xaa₃ is a basic amino acid;
Each occurrence of Yaa₁, Yaa₂, Yaa₃, Yaa₄, ..., Yaaₙ, Yaaₙ₊₁ is independently selected from none, one or more different or identical amino acids;
n2, n3, n4, ..., n all are an integer above 0, but they are not 0 at the same time.

12. The use of the recombinant collagen protein described in claim 1 in the preparation of products for regulating cell adhesion, proliferation, and differentiation.

13. The use of the recombinant collagen protein described in claim 1 in the preparation of products for cartilage repair matrix.

14. A cartilage repair matrix, **characterized in that** it comprises a polymer and/or a derivative thereof, the recombinant collagen protein as claimed in any one of claims 1 to 7; the stated polymer comprises natural polymers and synthetic polymers.

15. The cartilage repair matrix as claimed in claim 14, **characterized in that** the stated natural polymer is selected at least from any one of hyaluronic acid, chitosan, alginic acid, and cellulose; the stated synthetic polymer is selected at least from any one of PLA, PGA, PLCL, and PVA.

16. The preparation method of the cartilage repair matrix as claimed in claim 15, **characterized in that** it comprises the steps of:
(1) Dissolve the polymer derivative in a buffer solution, added with EDC/sNHS solution for reaction;
(2) Add the recombinant collagen protein stated in claim 1 to the reaction system of step (1) for reaction, and dialyze and dry the solution obtained after reaction to obtain the composite matrix;
(3) Dissolve the composite matrix, added with a photoinitiator solution to obtain the cartilage repair matrix.

17. The preparation method of the cartilage repair matrix as claimed in claim 16, **characterized in that** the polymer derivative described in step (1) is hyaluronic acid modified using methacrylic anhydride; the molecular weight of the modified hyaluronic acid is within 100 to 1500 kDa; the grafting degree of the stated modified hyaluronic acid is within 20 to 60%; the pH value of the mixed solution after the addition of the EDC/sNHS solution is within 4 to 5; after the addition of EDC and sNHS, the concentration of sNHS is within 0.5 to 5 mM and the concentration of EDC is within 0.5 to 5 mM; the pH value of the mixed solution after the reaction is within 7 to 8, and the reaction time is within 2 to 5h;
In step (2), the mass ratio of recombinant collagen protein: polymer derivative is 0.5-30: 2-10; the reaction time is within 5 to 15h; the reacted solution is loaded into a dialysis bag with a molecular weight cut-off of 8,000 to 14,000, dialyzed with deionized water for 2 to 4 days, lyophilized to give the composite matrix;
In step (3), the concentration of the composite matrix after the addition of photoinitiator is within 2 to 30 mg/ml.

18. The preparation method of the cartilage repair matrix as claimed in claim 17, **characterized in that** in step (1), the molecular weight of the modified hyaluronic acid is within 300 to 600 kDa; the degree of modified grafting of hyaluronic acid is within 30 to 50%;
In step (2), the mass ratio of recombinant collagen protein: polymer derivative is within 0.5 to 20:6, and the reaction time is within 8 to 12h;
In step (3), the concentration of the composite matrix after the addition of photoinitiator is within 5 to 20 mg/ml.

19. A preparation method of a cartilage repair hydrogel, **characterized in that** the cartilage repair matrix obtained by the method stated in claim 16 is mixed with chondrocytes or bone marrow mesenchymal stem cells, and injected into the site of cartilage damage, then they form into a cartilage repair hydrogel after light exposure.
